# DEMANDE DE BREVET EUROPEEN

(11) **EP 2 289 561 A1**
(43) Date de publication de la demande: **02.03.2011**
(21) Numéro de dépôt: 10179575.5
(22) Date de dépôt: 23.04.2004
(51) Int. Cl.: A61K 47/48, A61K 9/16, A61K 9/14

(54) **Procédé de préparation de complexes moleculaires**

(30) Priorité: 25.04.2003 FR 0305140
(62) Demande divisionnaire de: 04742568.1
(71) Demandeur: Pierre Fabre Medicament, 92100 Boulogne-Billancourt (FR)
(72) Inventeur: Lochard, Hubert, F-81600 BRENS (FR); Sauceau, Martial, F-81000 ALBI (FR); Freiss, Bernard, F-81100 CASTRES (FR)
(74) Mandataire: Ahner, Francis

(57) **Abrégé**

Complexes moléculaires solubles comprenant une ou plusieurs substances actives peu solubles dans un milieu aqueux incluses dans une ou plusieurs molécules hôtes, **caractérisés en ce qu**'ils sont susceptibles d'être obtenus par le procédé de préparation constitué par les étapes suivantes:
(a) mise en contact d'une ou plusieurs substances actives avec une ou plusieurs molécules hôtes,
(b) mise en oeuvre d'une étape de diffusion moléculaire par mise en contact, en mode statique, d'un fluide dense sous pression avec le mélange obtenu à l'étape (a) en présence d'un ou plusieurs agents de diffusion,
(c) récupération du complexe moléculaire ainsi formé.

## Description

La présente invention concerne un procédé de préparation de complexes moléculaires solubles par la technologie des fluides denses sous pression, en particulier celle du CO₂.

Les nouvelles molécules pharmaceutiques, à forte valeur ajoutée, sont dans 40% des cas insolubles ou peu solubles dans l'eau, ce qui nuit à leur biodisponibilité. L'augmentation de la surface spécifique des poudres permet d'améliorer leur vitesse de dissolution.

Or la biodisponibilité de principes actifs peut être considérablement augmentée si leur vitesse de dissolution est améliorée.

Dans les domaines pharmaceutique, cosmétique et nutraceutique, il existe un certain nombre de demandes de brevets, brevets et publications relatifs à la formation, en milieu sous pression, de complexes moléculaires d'une substance active dans un substrat enrobant. Néanmoins, la plupart des procédés décrits ne concerne pas l'objectif d'amélioration de la biodisponibilité, mais plutôt l'adsorption d'une substance active sur un substrat.

Bertucco et al. (Drugs encapsulation using a compressed gas antisolvent technique - Proceedings of the 4th Italian Conference on Supercitical Fluids and their Applications 1997, 327-334 - Ed. E. Reverch*on*) décrivent un procédé dans lequel, on met en suspension la substance active dans une solution de bio polymère jouant le rôle du support. Cette suspension, placée dans l'autoclave, est ensuite mise en présence de CO₂ supercritique pour la désolvater (extraction du solvant par fluide supercritique) et entraîner la complexation du support par sursaturation sur la substance active. Ce procédé est un procédé batch, dans lequel la substance active n'est pas précipitée par le fluide supercritique puisqu'elle est en suspension. La structure des particules de substance active est donc inchangée, ce qui ne contribue pas à améliorer sa dissolution dans un milieu aqueux.

Un procédé analogue est décrit par Benoît et al. dans leur demande de brevet WO98/13136.

Une autre technique de déposition d'un support, consiste à solubiliser ledit support dans le fluide supercritique, puis à faire précipiter ce support sur la substance active. Pour ce faire, la substance active et son support sont préalablement placés dans l'autoclave agité, et l'injection de CO₂ supercritique solubilise uniquement le support (ceci implique que le support soit soluble dans le fluide supercritique et que la substance active ne le soit pas), qui est précipité par modification de la pression et de la température au sein de l'autoclave. Dans ce cas, la structure initiale de la substance active reste inchangée, et il est difficile de maîtriser le ratio substance active/support obtenu dans le complexe précipité. Ce procédé batch est détaillé dans la demande de brevet EP 706 821 de Benoît et al.

Le procédé de microencapsulation décrit par Shine et Gelb dans leur demande de brevet WO98/15348 consiste en :
1. Mélanger une substance active avec un polymère d'encapsulation,
2. Liquéfier le polymère par passage d'un flux de fluide supercritique,
3. Dépressuriser rapidement de façon à solidifier le polymère autour de la substance active.

Ce procédé n'est applicable qu'avec une substance active et un polymère insolubles dans le fluide supercritique. De ce fait, la substance active conserve sa structure d'origine, ce qui ne contribue pas à améliorer sa biodisponibilité.

Dans la demande de brevet FR2798863 de Perrut et Majewski, la substance active (kava-kava, curcuma, mélange de poivre noir et de paprika doux), préalablement extraite par fluide supercritique, est précipitée dans un autoclave contenant un support poreux. Le milieu poreux étudié est la maltodextrine. Les auteurs revendiquent un procédé d'adsorption de la substance active sur un milieu poreux, et non pas un phénomène de diffusion de la substance active dans la molécule-hôte, permettant d'améliorer la dissolution du complexe moléculaire obtenu.

Un procédé d'imprégnation d'actifs pharmaceutiques est revendiqué dans la demande de brevet WO 99/25322 de Carli et al.. Il se décompose de la manière suivante :
1. Solubilisation du principe actif par un fluide supercritique,
2. Mise en contact du fluide supercritique contenant le principe actif avec le polymère réticulé,
3. Imprégnation du polymère réticulé en mode statique ou dynamique,
4. Elimination du fluide supercritique.

Seules des substances actives solubles dans le fluide supercritique peuvent être préparées par ce procédé, puisque la première étape consiste en l'extraction du principe actif par le fluide supercritique. Par ailleurs, le procédé n'est pas un procédé d'inclusion mais d'imprégnation sur un support, et aucun résultat n'est donné concernant l'amélioration de la dissolution dans un milieu aqueux du principe actif ainsi préparé.

Van Hees et al. (Application of supercritical carbon dioxide for the preparation of a Piroxicam-β-cyclodextrin inclusion compound, Pharmaceutical Research, Vol. 16, N°12, 1999**)** décrivent dans leur publication un procédé d'inclusion de Piroxicam dans les β-cyclodextrines par CO₂ supercritique. Le piroxicam étant peu soluble dans l'eau, son inclusion dans les β-cyclodextrines doit permettre d'augmenter sa solubilité dans l'eau. Le procédé consiste à placer un mélange de piroxicam et de β-cyclodextrines dans un réacteur, laissé en mode statique. Après dépressurisation le mélange obtenu est broyé et homogénéisé avant caractérisation par :
- DSC (Differential Scanning Calorimetry),
- mesure de solubilité dans l'acétonitrile et comparaison avec la solubilité du piroxicam seul, et
- méthodes spectroscopiques.

L'analyse par DSC permet de conclure quant à la complexation du piroxicam avec la β-cyclodextrine.

Kamihira M. et al.(J. of Fermentation and Bioengineering, Vol. 69, N°6, 350-353, 1990) décrivent un procédé d'extraction de composés aromatiques volatiles, et de piégeage par inclusion dans les cyclodextrines. Le géraniol et l'huile de moutarde sont ainsi extraits par un fluide supercritique, puis vaporisés en mode dynamique dans un second réacteur contenant des cyclodextrines hydratées. L'influence des différents paramètres est étudiée par mesure du taux d'inclusion des composés aromatiques dans les cyclodextrines. L'étape d'inclusion est réalisée en mode dynamique et non statique. Par ailleurs, l'application revendiquée par les auteurs est toute autre puisqu'il s'agit de fixation de molécules volatiles par inclusion. Enfin, ce procédé n'est pas mis en oeuvre avec des fluides supercritiques, mais avec des gaz sous pression.

Enfin, la demande internationale WO 03/030867, déposée au nom de PIERRE FABRE MEDICAMENT vise un procédé de préparation de composés d'interaction d'un dérivé d'anilide avec un support poreux, comprenant obligatoirement les étapes suivantes:
a) mélanger le dérivé d'anilide généré par fluide supercritique et la quantité déterminée de support poreux,
b) mettre en oeuvre une étape de diffusion moléculaire par mise en contact en mode statique d'un fluide supercritique avec le mélange obtenu à l'étape a),
c) laver le composé d'interaction obtenu à l'étape b) par un flux de fluide supercritique, et
d) récupérer les particules du composé d'interaction ainsi formé.

Il convient de noter l'étape c) de lavage réalisée en milieu supercritique est essentielle puisqu'elle permet d'éliminer les solvants résiduels et participe à l'amélioration de la solubilité du principe actif.

Toutefois l'ensemble de ces procédés semble difficilement utilisable pour préparer des complexes d'inclusion à l'échelle industrielle.

De façon surprenante, les inventeurs de la présente demande ont découvert qu'un procédé comprenant une étape de diffusion moléculaire par un fluide dense sous pression en mode statique et dépourvu de l'étape de lavage subséquente à l'aide d'un fluide supercritique, améliorait significativement le taux d'inclusion en fonction de la quantité d'un agent de diffusion ajoutée au milieu.

Ainsi, la présente invention concerne un procédé de préparation de complexes moléculaires solubles comprenant une ou plusieurs substances actives peu soluble dans un milieu aqueux incluses dans une ou plusieurs molécules hôtes, **caractérisé en ce qu**'il est limitativement constitué par les étapes suivantes :
(a) mise en contact d'une ou plusieurs substances actives avec une ou plusieurs molécules hôtes,
(b) mise en oeuvre d'une étape de diffusion moléculaire par mise en contact, en mode statique, d'un fluide dense sous pression avec le mélange obtenu à l'étape (a) en présence d'un ou plusieurs agents de diffusion,
(c) récupération du complexe moléculaire ainsi formé.

Par «fluide dense sous pression», on entend au sens de la présente invention tout fluide utilisé à une température ou une pression supérieure à leur valeur critique. Avantageusement il s'agit du CO₂ pur ou en mélange avec un solvant organique classiquement utilisé par l'homme du métier.

Par « substance active peu soluble dans un milieu aqueux », on entend au sens de la présente invention toute substance active peu ou pas soluble dans un milieu aqueux et ayant en particulier une solubilité inférieure à au moins 20 µg/ml. En particulier il peut s'agir d'un actif pharmaceutique, (on peut citer à titre d'exemple les analgésiques, les antipyrétiques, l'aspirine et ses dérivés, les antibiotiques, les anti-inflammatoires, les antiulcéreux, les antihypertenseurs, les neuroleptiques, les antidépresseurs, les oligonucléotides présentant une activité thérapeutique, les peptides présentant une activité thérapeutique et les protéines présentant une activité thérapeutique), cosmétique ou nutraceutique. Avantageusement il s'agit d'une substance active choisie dans le groupe comprenant les dérivés d'anilide, les dérivés d'épipodophyllotoxine, le minoxidil, le piroxicam, l'acide valérique, l'acide octanoïque, l'acide laurique, l'acide stéarique, l'acide tiaprofénique, l'oméprazole et l'éflucimibe.

Par « molécule hôte », on entend au sens de la présente invention toute substance apte à capter des substances actives. Avantageusement la molécule hôte est choisie dans le groupe constitué par les polyoses et les oses, notamment les cyclodextrines et leur mélange. De façon avantageuse, il s'agit de la β-cyclodextrine, de la méthyl-β-cyclodextrine, de la γ-cyclodextrine ou de l'hydroxypropyl-β-cyclodextrine.

Par « agent de diffusion », on entend au sens de la présente invention n'importe quel solvant favorisant une interaction de la substance active avec la molécule-hôte. Avantageusement, cet agent de diffusion est choisi dans le groupe constitué par les alcools, les cétones, les éthers, les esters et l'eau avec ou sans agent surfactant et leurs mélanges. De façon encore plus avantageuse, il s'agit de l'eau.

Par « mode statique » on entend au sens de la présente invention une réaction ou un procédé dans lequel tous les réactifs sont mis simultanément en présence et où on laisse la réaction se dérouler. Par exemple, dans l'étape (b) de la présente invention, on met dans un autoclave la ou les substance(s) active(s), de l'eau et du CO₂ supercritique et on laisse réagir pendant plusieurs heures. La masse de produit n'évolue pas durant la réaction. A l'inverse, en mode dynamique, les réactifs sont apportés au fur et à mesure de l'évolution de la réaction ou de la production. Souvent dans le cadre d'un mode dynamique, il y a circulation d'un fluide ou agitation. La masse de produit évolue durant la production.

La substance active et la molécule-hôte sont introduites sous forme solide ou liquide dans un récipient dans lequel est injecté le fluide dense sous pression et l'agent de diffusion dans des proportions judicieusement choisies. Les conditions de pression et de température ainsi que la durée du traitement sont définies, par toute méthode appropriée, en fonction de la nature de la ou des substances actives et du ou des molécules-hôtes.

De façon avantageuse, l'étape (b) de diffusion moléculaire du procédé selon la présente invention est réalisée sous agitation.

L'agent de diffusion peut être ajouté en continu ou en discontinu dans une quantité comprise entre 1 et 50% en masse, de préférence entre 20 et 25% en masse.

Le temps nécessaire à la diffusion moléculaire de l'étape (b) est déterminé par toute méthode appropriée. Cette étape (b) peut être réitérée autant de fois que souhaitée pour obtenir une vitesse de dissolution satisfaisante. Avantageusement, l'étape (b) dure entre environ 2 et 16 heures.

Les conditions de pression et de température de l'étape (b) sont choisies de façon à favoriser la diffusion moléculaire. Avantageusement la pression du fluide supercritique est comprise entre 5 MPa et 40 MPa et la température entre 0 et 120°C.

Avantageusement l'étape (b) du procédé selon la présente invention est mise en oeuvre dans un réacteur fermé, en particulier un autoclave.

Le procédé peut être mis en oeuvre en batch ou en continu. De façon avantageuse le procédé selon la présente invention est réalisé en batch.

La présente invention concerne également les complexes moléculaires solubles comprenant une ou plusieurs substance(s) active(s) peu soluble(s) dans un milieu aqueux incluses dans une ou plusieurs molécules hôtes, **caractérisés en ce qu**'ils ont susceptibles d'être obtenus par le procédé selon la présente invention.

La mise en oeuvre de l'étape de diffusion moléculaire en milieu dense sou pression en présence d'un agent de diffusion permet une forte interaction des particules de substance active avec la molécule hôte, ce qui favorise la dissolution en milieu aqueux, laquelle est multipliée par environ 100 par le procédé selon la présente invention.

Les exemples suivants de mise en oeuvre du procédé sont donnés à titre indicatif non limitatif.

### Exemple 1: Minoxidil (substance active) et la y-cyclodextrine (molécule-hôte)

### 1.1. Méthodes d'évaluation du taux d'inclusion

Le taux d'inclusion de la substance active dans la molécule-hôte est évalué par analyse thermique différentielle (*Differential Scanning Calorimetry*). On applique une rampe de température sous flux d'azote au produit à tester, à l'aide d'un appareil DSC 7 Perkin Elmer.

Le rendement de complexation est évalué par mesure de la réduction (ou disparition) du pic thermique relatif au principe actif resté libre.

### 1.2. Sans ajout d'agent de diffusion

Une mole de minoxidil et deux moles de γ-cyclodextrine sont introduits dans un réacteur. Le dioxyde de carbone est par la suite introduit dans le réacteur sous une pression de 15 MPa et sous une température de 80 °C. L'ensemble est maintenu dans ces conditions opératoires pendant une durée de deux heures.

Après détente du milieu, le taux d'inclusion est mesuré sur la poudre collectée et s'avère égal à 0%. Aucune inclusion de la substance active n'est observable dans la molécule-hôte.

### 1.3. Avec ajout d'agent de diffusion

Une mole de minoxidil et deux moles de γ-cyclodextrine sont introduits dans un réacteur, ainsi que 12,1% massique d'agent de diffusion (eau). Le dioxyde de carbone est par la suite introduit dans le réacteur sous une pression de 15 MPa et sous une température de 80°C. L'ensemble est maintenu dans ces conditions opératoires pendant une durée de deux heures.

Après détente du milieu, le taux d'inclusion est mesuré sur la poudre collectée et s'avère égal à 45%.

Un second essai a été réalisé dans les mêmes conditions opératoires que précédemment, à l'unique différence que la quantité d'agent de diffusion ajouté a été portée à 23,1 %.

Après détente du milieu, le taux d'inclusion est mesuré sur la poudre collectée et s'avère égal à 62%.

### Exemple 2: Minoxidil (substance active) et la méthyl-β-cyclodextrine (molécule-hôte)

### 2.1. Sans ajout d'agent de diffusion

Une mole de minoxidil et deux moles de méthyl-β-cyclodextrine sont introduits dans un réacteur. Le dioxyde de carbone est par la suite introduit dans le réacteur sous une pression de 15 MPa et sous une température de 80 °C. L'ensemble est maintenu dans ces conditions opératoires pendant une durée de deux heures.

Après détente du milieu, le taux d'inclusion est mesuré sur la poudre collectée et s'avère égal à 17%.

### 2.2. Avec ajout d'agent de diffusion

Une mole de minoxidil et deux moles de méthyl-β-cyclodextrine sont introduits dans un réacteur, ainsi que 8,4% massique d'agent de diffusion (eau). Le dioxyde de carbone est par la suite introduit dans le réacteur sous une pression de 15 MPa et sous une température de 80 °C. L'ensemble est maintenu dans ces conditions opératoires pendant une durée de deux heures.

Après détente du milieu, le taux d'inclusion est mesuré sur la poudre collectée et s'avère égal à 60%.

### Exemple 3: Piroxicam (substance active) et la β-cyclodextrine (molécule-hôte)

### 3.1. Sans ajout d'agent de diffusion

Une mole de piroxicam et deux moles de β-cyclodextrine sont introduits dans un réacteur. Le dioxyde de carbone est par la suite introduit dans le réacteur sous une pression de 15 MPa et sous une température de 150 °C. L'ensemble est maintenu dans ces conditions opératoires pendant une durée de deux heures.

Après détente du milieu, le taux d'inclusion est mesuré sur la poudre collectée et s'avère égal à 0%. Aucune inclusion de la substance active n'est observable dans la molécule-hôte.

### 3.2. Avec ajout d'agent de diffusion

Une mole de piroxicam et deux moles de β-cyclodextrine sont introduits dans un réacteur, ainsi que 11,8% massique d'agent de diffusion (eau). Le dioxyde de carbone est par la suite introduit dans le réacteur sous une pression de 15 MPa et sous une température de 150 °C. L'ensemble est maintenu dans ces conditions opératoires pendant une durée de deux heures.

Après détente du milieu, le taux d'inclusion est mesuré sur la poudre collectée et s'avère égal à 50%.

Un second essai a été réalisé dans les mêmes conditions opératoires que précédemment, à l'unique différence que la quantité d'agent de diffusion ajouté a été portée à 19,8%.

Après détente du milieu, le taux d'inclusion est mesuré sur la poudre collectée et s'avère égal à 92%.

### Exemple 4: Piroxicam (substance active) et la y-cyclodextrine (molécule-hôte)

### 4.1. Sans ajout d'agent de diffusion

Une mole de piroxicam et deux moles de γ-cyclodextrine sont introduits dans un réacteur. Le dioxyde de carbone est par la suite introduit dans le réacteur sous une pression de 15 MPa et sous une température de 150 °C. L'ensemble est maintenu dans ces conditions opératoires pendant une durée de deux heures.

Après détente du milieu, le taux d'inclusion est mesuré sur la poudre collectée et s'avère égal à 0%. Aucune inclusion de la substance active n'est observable dans la molécule-hôte.

### 4.2. Avec ajout d'agent de diffusion

Une mole de piroxicam et deux moles de γ-cyclodextrine sont introduits dans un réacteur, ainsi que 22% massique d'agent de diffusion (eau). Le dioxyde de carbone est par la suite introduit dans le réacteur sous une pression de 15 MPa et sous une température de 150 °C. L'ensemble est maintenu dans ces conditions opératoires pendant une durée de deux heures.

Après détente du milieu, le taux d'inclusion est mesuré sur la poudre collectée et s'avère égal à 28%.

### Exemple 5: Acide tiaprofénique (substance active) et la γ-cyclodextrine (molécule-hôte)

### 5.1. Sans ajout d'agent de diffusion

Une mole d'acide tiaprofénique et deux moles de γ-cyclodextrine sont introduits dans un réacteur. Le dioxyde de carbone est par la suite introduit dans le réacteur sous une pression de 15 MPa et sous une température de 50°C. L'ensemble est maintenu dans ces conditions opératoires pendant une durée de deux heures.

Après détente du milieu, le taux d'inclusion est mesuré sur la poudre collectée et s'avère égal à 19%.

### 5.2. Avec ajout d'agent de diffusion

Une mole d'acide tiaprofénique et deux moles de γ-cyclodextrine sont introduits dans un réacteur, ainsi que 20,5% massique d'agent de diffusion (eau). Le dioxyde de carbone est par la suite introduit dans le réacteur sous une pression de 15 MPa et sous une température de 50°C. L'ensemble est maintenu dans ces conditions opératoires pendant une durée de deux heures. ² Après détente du milieu, le taux d'inclusion est mesuré sur la poudre collectée et s'avère égal à 100%. L'inclusion de la substance active dans la molécule-hôte semble complète dans ce cas.

### Exemple 6: Oméprazole (substance active) et la γ-cyclodextrine (molécule-hôte)

### 6.1. Sans ajout d'agent de diffusion

Une mole d'oméprazole et deux moles de γ-cyclodextrine sont introduits dans un réacteur. Le dioxyde de carbone est par la suite introduit dans le réacteur sous une pression de 15 MPa et sous une température de 100°C. L'ensemble est maintenu dans ces conditions opératoires pendant une durée de deux heures.

Après détente du milieu, le taux d'inclusion est mesuré sur la poudre collectée et s'avère égal à 2%. Sans ajout d'agent de diffusion, l'inclusion de la substance active dans la molécule-hôte semble très faible.

### 6.2. Avec ajout d'agent de diffusion

Une mole d'oméprazole et deux moles de γ-cyclodextrine sont introduits dans un réacteur, ainsi que 20,7% massique d'agent de diffusion. Le dioxyde de carbone est par la suite introduit dans le réacteur sous une pression de 15 MPa et sous une température de 100°C. L'ensemble est maintenu dans ces conditions opératoires pendant une durée de deux heures.

Après détente du milieu, le taux d'inclusion est mesuré sur la poudre collectée et s'avère égal à 66%.

### Exemple 7: Eflucimibe (substance active) et la γ-cyclodextrine (molécule-hôte)

### 7.1. Sans ajout d'agent de diffusion

Une mole d'eflucimibe et deux moles de γ-cyclodextrine sont introduits dans un réacteur. Le dioxyde de carbone est par la suite introduit dans le réacteur sous une pression de 30 MPa et sous une température de 100°C. L'ensemble est maintenu sous ces conditions opératoires pendant une durée de seize heures.

Après détente du milieu, le taux d'inclusion est mesuré sur la poudre collectée et s'avère égal à 0 %. Sans ajout d'agent de diffusion, l'inclusion de la substance active dans la molécule-hôte est nulle.

### 7.2. Avec ajout d'agent de diffusion

Une mole d'eflucimibe et deux moles de γ-cyclodextrine sont introduits dans un réacteur, ainsi que 25 % massique d'agent de diffusion (eau). Le dioxyde de carbone est par la suite introduit dans le réacteur sous une pression de 30 MPa et sous une température de 100 °C. L'ensemble est maintenu sous ces conditions opératoires pendant une durée de deux heures.

Après détente du milieu, le taux d'inclusion est mesuré sur la poudre collectée et s'avère égal à 60 %.

### 7.3. Test de dissolution

Un test de dissolution a été effectué sur les produits obtenus aux exemples 7.1 et 7.2.

Après 2 heures d'agitation dans une solution de laurylsulfate de sodium à 5%, la quantité d'eflucimibe solubilisée à partir de la poudre collectée dans l'exemple 7.1 est de 24µg/ml au lieu de 22µg/ml pour le mélange initial.

Après 2 heures d'agitation dans une solution de laurylsulfate de sodium à 5%, la quantité d'eflucimibe solubilisée à partir de la poudre collectée dans l'exemple 7. 2 est de 160µg/ml au lieu de 22µg/ml pour le mélange initial.

L'ensemble des résultats donnés ci-dessus montre l'importance majeure de l'ajout d'un agent de diffusion afin d'améliorer le taux d'inclusion de la substance active dans la molécule-hôte et par conséquent la dissolution dans l'eau.

## Revendications

1. Complexes moléculaires solubles comprenant une ou plusieurs substances actives peu solubles dans un milieu aqueux incluses dans une ou plusieurs molécules hôtes, **caractérisés en ce qu'**ils sont susceptibles d'être obtenus par le procédé de préparation constitué par les étapes suivantes:
(a)mise en contact d'une ou plusieurs substances actives avec une ou plusieurs molécules hôtes,
(b)mise en oeuvre d'une étape de diffusion moléculaire par mise en contact, en mode statique, d'un fluide dense sous pression avec le mélange obtenu à l'étape (a) en présence d'un ou plusieurs agents de diffusion,
(c)récupération du complexe moléculaire ainsi formé.

2. Complexe selon la revendication 1, **caractérisé en ce que** le fluide dense sous pression est du CO₂.

3. Complexe selon l'une quelconque des revendications 1 et 2, **caractérisé en ce que** la substance active est un actif pharmaceutique, de préférence choisi dans le groupe comprenant les analgésiques, les antipyrétiques, l'aspirine et ses dérivés, les antibiotiques, les anti-inflammatoires, les antiulcéreux, les antihypertenseurs, les neuroleptiques, les antidépresseurs, les oligonucléotides présentant une activité thérapeutique, les peptides présentant une activité thérapeutique et les protéines présentant une activité thérapeutique, cosmétique ou nutraceutique.

4. Complexe selon la revendication 3, **caractérisé en ce que** la substance active est choisie dans le groupe comprenant les dérivés d'anilide, les dérivés d'épipodophyllotoxine, le minoxidil, le piroxicam, l'acide valérique, l'acide octanoïque, l'acide laurique, l'acide stéarique, l'acide tiaprofénique, l'oméprazole et l'éflucimibe.

5. Complexe selon l'une quelconque des revendications 1 à 4, **caractérisé en ce que** la molécule hôte est choisie dans le groupe constitué par les polyoses et les oses, de préférence parmi les cyclodextrines et leur mélange.

6. Complexe selon l'une quelconque des revendications 1 à 5, **caractérisé en ce que** lequel l'agent de diffusion est choisi dans le groupe constitué par les alcools, les cétones, les éthers, les esters et l'eau avec ou sans agent surfactant et leurs mélanges.

7. Complexe selon la revendication 6, **caractérisé en ce que** l'agent de diffusion est l'eau.

8. Complexe selon l'une quelconque des revendications 1 à 7, **caractérisé en ce que** l'étape (b) de diffusion moléculaire est réalisée sous agitation.

9. Complexe selon l'une quelconque des revendications 1 à 8, **caractérisé en ce que** l'agent de diffusion est ajouté en continu ou en discontinu dans une quantité comprise entre 1 et 50 % en masse, de préférence entre 20 et 25 % en masse.

10. Complexe selon l'une quelconque des revendications 1 à 9, **caractérisé en ce que** la pression du fluide supercritique est comprise entre 5 MPa et 40 MPa et la température entre 0 et 120°C.
